# EUROPÄISCHE PATENTSCHRIFT

(11) **EP 1 140 890 B1**
(45) Veröffentlichungstag und Bekanntmachung des Hinweises auf die Patenterteilung: **26.02.2003**
(21) Anmeldenummer: 99965422.1
(22) Anmeldetag: 01.12.1999
(51) Int. Cl.: C07D 311/22, C07D 311/58, C07D 405/12

(54) **CHROMANDERIVATE**
CHROMAN DERIVATIVES
DERIVES DE CHROMANE

(30) Priorität: 17.12.1998 DE 19858341
(43) Veröffentlichungstag der Anmeldung: 10.10.2001
(73) Patentinhaber: MERCK PATENT GmbH, 64293 Darmstadt (DE)
(72) Erfinder: BOKEL, Heinz-Hermann, D-64287 Darmstadt (DE); MACKERT, Peter, D-63329 Egelsbach (DE); MÜRMANN, Christoph, D-64354 Reinheim (DE); SCHWEICKERT, Norbert, D-64342 Seeheim-Jugenheim (DE)
(86) Internationale Anmeldenummer: EP9909333
(87) Internationale Veröffentlichungsnummer: WO00035901

(56) Entgegenhaltungen:
- EP-A- 0 540 914
- EP-A- 0 707 007

## Beschreibung

Die Erfindung betrifft Chromanderivate der Formel I 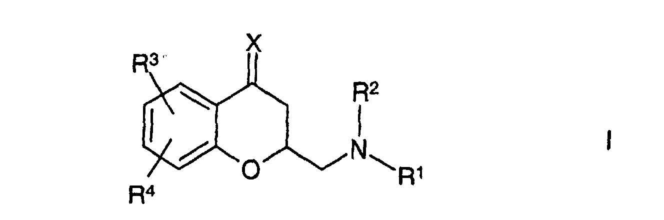 worin
- R¹: Acyl mit 1-6 C-Atomen, -CO-R⁵, Phenylacetyl, Phenoxyacetyl, Methoxycarbonyl, Ethoxycarbonyl, 2,2,2-Trichlorethoxycarbonyl, tert.-Butoxycarbonyl, 2-lodethoxycarbonyl, Carbobenzoxycarbonyl, 4-Methoxybenzyloxycarbonyl, 9-Fluorenylmethoxycarbonyl oder 4-Methoxy-2,3,6-trimethylphenyl-sulfonyl,
- R²: H oder Alkyl mit 1-6 C-Atomen,
- R³, R⁴: jeweils unabhängig voneinander H, Alkyl mit 1-6 C-Atomen, CN, Hal oder COOR²,
- R⁵: unsubstituiertes oder ein- oder zweimal durch Alkyl mit 1-6 C-Atomen, OR² oder Hal substituiertes Phenyl,
- X: H,H oder O,
- Hal: F, Cl, Br oder I
bedeuten,
sowie deren Salze,
wobei N-(Chroman-2-ylmethyl)-cyclopropancarbonsäureamid ausgeschlossen ist.
Gegenstand der Erfindung sind auch die optisch aktiven Formen, die Racemate, die Enantiomeren sowie die Hydrate und Solvate, z.B. Alkoholate, dieser Verbindungen.

Ähnliche Verbindungen sind aus EP 0 707 007 und EP 0 540 914 bekannt.

Der Erfindung lag die Aufgabe zugrunde, neue Verbindungen zu finden, die insbesondere als Zwischenprodukte bei der Synthese von Arzneimitteln verwendet werden können.

Es wurde gefunden, daß die Verbindungen der Formel I und ihre Salze wichtige Zwischenprodukte zur Herstellung von Arzneimitteln darstellen, insbesondere von solchen, die beispielsweise Wirkungen auf das Zentralnervensystem zeigen.

Gegenstand der Erfindung sind die Chromanderivate der Formel I und ihre Salze.

Vor- und nachstehend haben die Reste R¹, R², R³, R⁴, R⁵ und X, die bei den Formeln I und II angegebenen Bedeutungen, falls nicht ausdrücklich etwas anderes angegeben ist.

In den vorstehenden Formeln hat Alkyl 1 bis 6, vorzugsweise 1, 2, 3 oder 4 C-Atome. Alkyl bedeutet vorzugsweise Methyl oder Ethyl, weiterhin Propyl, Isopropyl, ferner auch Butyl, Isobutyl, sek.-Butyl oder tert.-Butyl. Acyl hat 1 bis 6, vorzugsweise 1, 2, 3 oder 4 C-Atome. Acyl bedeutet insbesondere Acetyl, Propionyl oder Butyryl.
R² bedeutet vorzugsweise H, ferner auch Methyl, Ethyl oder Propyl.
R³ und R⁴ bedeuten vorzugsweise H.
R⁵ bedeutet vorzugsweise z.B. Phenyl, o-, m- oder p-Tolyl, o-, m- oder p-Hydroxyphenyl, o-, m- oder p-Methoxyphenyl, o-, m- oder p-Fluorophenyl. Der Rest R¹ bedeutet Acyl, -CO-R⁵ oder aber eine an sich bekannte Aminoschutzgruppe, besonders bevorzugt ist Acetyl.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren und den vorliegenden Verbindungen in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, BOC (tert.-Butoxycarbonyl), 2-lodethoxycarbonyl; Aralkyloxycarbonyl wie CBZ (Carbobenzoxycarbonyl, auch "Z" genannt), 4-Methoxybenzyloxycarbonyl, FMOC (9-Fluorenylmethoxycarbonyl); Arylsulfonyl wie Mtr (4-Methoxy-2,3,6-trimethylphenyl-sulfonyl). Bevorzugte Aminoschutzgruppen sind BOC und Mtr, ferner CBZ oder FMOC.

Die Verbindungen der Formel I können ein oder mehrere chirale Zentren besitzen und daher in verschiedenen stereoisomeren Formen vorkommen. Die Formel I umschließt alle diese Formen.

Gegenstand der Erfindung ist ferner ein Verfahren zur Herstellung von Chromanderivaten der Formel I nach Anspruch 1 sowie von deren Salzen, worin X O bedeutet,
dadurch gekennzeichnet, daß man eine Verbindung der Formel II 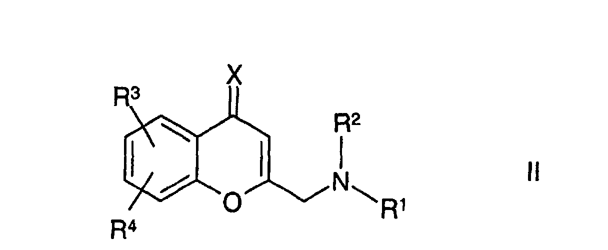 worin R¹, R², R³, R⁴ die in Anspruch 1 angegebenen Bedeutungen haben und X O bedeutet,
mit Hilfe eines enantiomerenangereicherten Katalysators hydriert.

Gegenstand der Erfindung ist auch ein Verfahren zur Herstellung von Chromanderivaten der Formel I nach Anspruch 1 sowie von deren Salzen, worin X H,H bedeutet,
dadurch gekennzeichnet, daß man eine Verbindung der Formel II 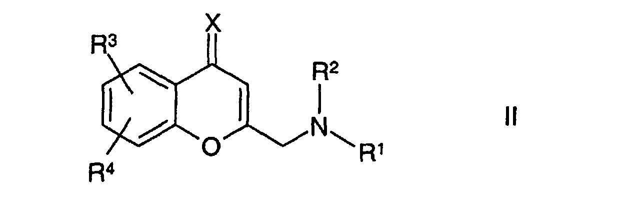 worin R¹, R², R³, R⁴ die in Anspruch 1 angegebenen Bedeutungen haben und X O bedeutet,
mit Hilfe eines enantiomerenangereicherten Katalysators hydriert,
und anschließend wie üblich reduziert.

Insbesondere wurde gefunden, daß (2-Acetylaminomethyl)-chromen-4-on mit verschiedenen enantiomerenreinen Rhodium-Diphosphan-Komplexen zu enantiomerenangereichertem (2-Acetylaminomethyl)-chroman-4-on hydriert werden kann.

Gegenstand der Erfindung ist auch ein Verfahren zur Herstellung von Chromanderivaten der Formel I, dadurch gekennzeichnet, daß es sich bei dem enantiomerenangereicherten Katalysator um einen Übergangsmetallkomplex handelt.

Besonders bevorzugt handelt es sich bei dem Katalysator um einen Übergangsmetallkomplex, enthaltend ein Metall ausgewählt aus der Gruppe Rhodium, Iridium, Ruthenium und Palladium.

Gegenstand der Erfindung ist ferner ein Verfahren zur Herstellung von Chromanderivaten der Formel I dadurch gekennzeichnet, daß es sich bei dem Katalysator um einen Übergangsmetallkomplex handelt, worin das Übergangsmetall mit einem chiralen Diphosphanliganden komplexiert ist.

Die nachstehenden Liganden sind beispielhaft zu nennen:

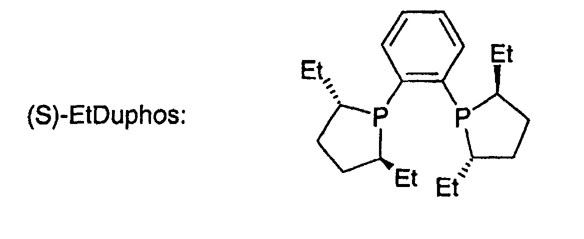

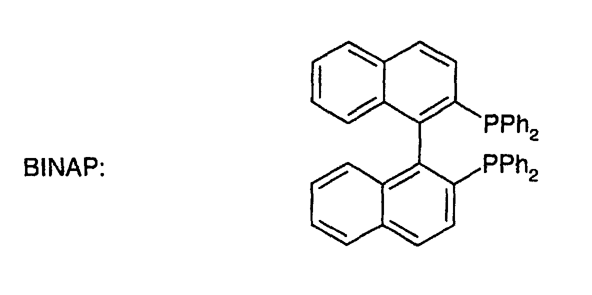

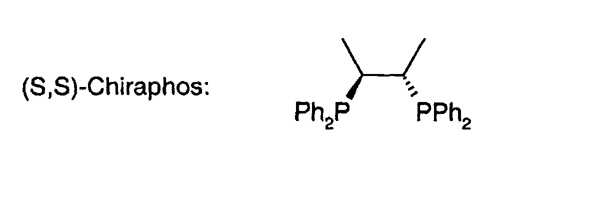

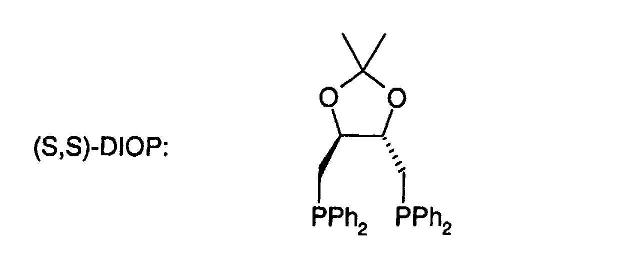

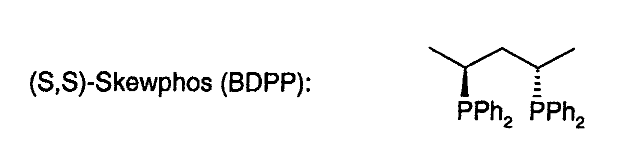

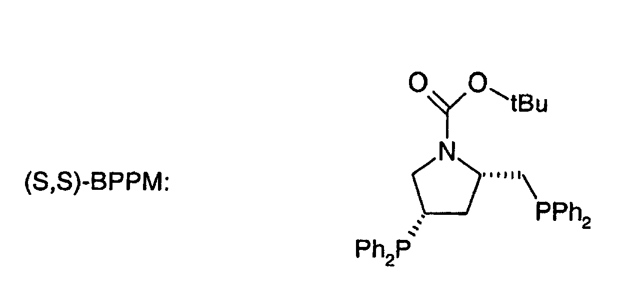

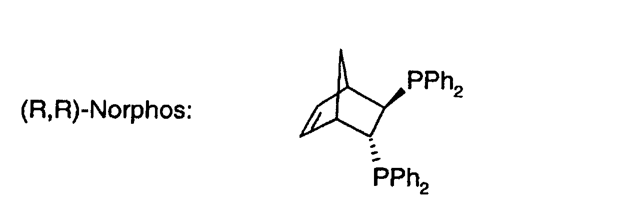

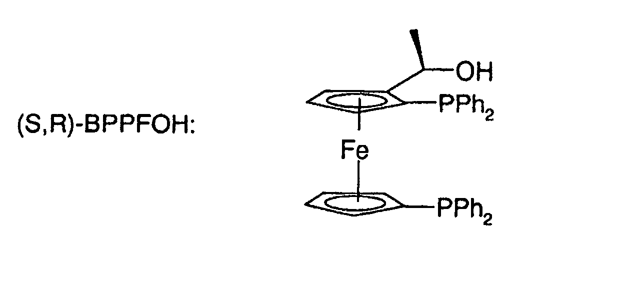

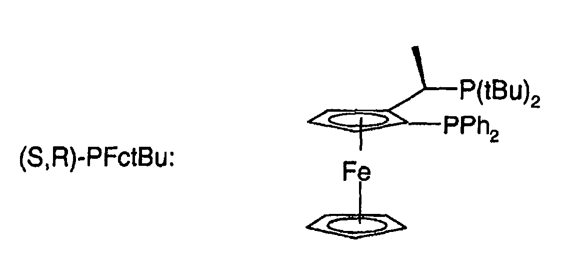

Je nach Wahl des (R)- oder (S)-Enantiomeren des Liganden im Katalysator wird das (R)- oder (S)-Enantiomere im Überschuß erhalten.

Als Precursor für die chiralen Liganden dienen Verbindungen wie z.B. Rh(COD)₂OTf (Rhodium-cyclooctadien-triflat), [Rh(COD)Cl]₂, Rh(COD)₂BF₄, [Ir(COD)Cl]₂, Ir(COD)₂BF₄ oder [Ru(COD)Cl₂]ₓ.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel II sind zum Teil bekannt.
So kennt man z.B. die Verbindung 2-(Acetylamino-methyl)-chromen-4-on (Beilstein RN 178696) aus Schmutz et al., Helv. Chim. Acta; **36,** 1953; 620, 623, 625.

Die nicht bekannten Verbindungen können leicht analog zu den bekannten Verbindungen hergestellt werden.
Die Umwandlung einer Verbindung der Formel II, in der X O bedeutet, in eine Verbindung der Formel I, in der X O bedeutet, erfolgt erfindungsgemäß mit Wasserstoffgas mit Hilfe eines enantiomerenangereicherten Katalysators in einem inerten Lösungsmittel wie beispielsweise Methanol oder Ethanol.

Als inerte Lösungsmittel eignen sich weiterhin z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Nitroverbindungen wie Nitromethan oder Nitrobenzol; Ester wie Ethylacetat, gegebenenfalls auch Gemische der genannten Lösungsmittel untereinander oder Gemische mit Wasser.

Die Reaktionszeit der enantioselektiven Hydrierung liegt je nach den angewendeten Bedingungen zwischen einigen Minuten und 14 Tagen, die Reaktionstemperatur zwischen 0 und 150°, normalerweise zwischen 20 und 130°.
Üblicherweise liegt das Verhältnis Katalysator/Substrat zwischen 1:2000 und 1:50, besonders bevorzugt bei 1:1000 bis 1:100. Die Reaktionszeit liegt dann z.B. zwischen 3 und 20 Stunden. Die Hydrierung wird unter 1-200 bar Wasserstoff durchgeführt, vorzugsweise bei 3-100 bar.

Die Umwandlung einer Verbindung der Formel II, in der X O bedeutet, in eine Verbindung der Formel I, in der X H,H bedeutet, erfolgt erfindungsgemäß mit Wasserstoffgas mit Hilfe eines enantiomerenangereicherten Katalysators in einem inerten Lösungsmittel wie Methanol oder Ethanol, wie oben beschrieben, gefolgt von einer Umwandlung der 4-Oxogruppe in eine Methylengruppe nach bekannten Bedingungen. Die Reduktion erfolgt vorzugsweise mit Wasserstoffgas unter Übergangsmetallkatalyse (beispielsweise durch Hydrierung an Raney-Nickel oder Pd-Kohle in einem inerten Lösungsmittel wie Methanol oder Ethanol).

Die Umwandlung von Verbindungen der Formel I, in der R³, R⁴ COOAlkyl bedeutet, in Verbindungen der Formel I, in der R³, R⁴ COOH bedeutet, erfolgt z.B. mit NaOH oder KOH in Wasser, Wasser-THF oder Wasser-Dioxan bei Temperaturen zwischen 0 und 100°.

Die Abspaltung eines Restes R¹ aus einer Verbindung der Formel I erfolgt - je nach der benutzten Schutzgruppe - z.B. mit starken Säuren, zweckmäßig mit TFA (Trifluoressigsäure) oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie Dimethylformamid, halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9 : 1. Die Reaktionstemperaturen liegen zweckmäßig zwischen etwa 0 und etwa 50°, vorzugsweise arbeitet man zwischen 15 und 30°.

Die Gruppe BOC wird bevorzugt mit TFA in Dichlormethan oder mit etwa 3 bis 5 n Salzsäure in Dioxan bei 15-30° abgespalten.
Die Abspaltung der Acetylgruppe erfolgt nach üblichen Methoden (P.J. Kocienski, *Protecting Groups,* Georg Thieme Verlag, Stuttgart, **1994**).

Hydrogenolytisch entfernbare Schutzgruppen (z.B. CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt.
Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Andererseits können Verbindungen der Formel I mit Basen (z.B. Natriumoder Kaliumhydroxid oder -carbonat) in die entsprechenden Metall-, insbesondere Alkalimetall- oder Erdalkalimetall-, oder in die entsprechenden Ammoniumsalze umgewandelt werden.

Gegenstand der Erfindung ist weiterhin die Verwendung der Verbindungen der Formel I als Zwischenprodukte zur Synthese von Arzneimitteln. Entsprechende Arzneimittel sind beispielsweise in EP 0 707 007 beschrieben.

Gegenstand der Erfindung ist dementsprechend insbesondere die Verwendung der Verbindungen der Formel I nach Anspruch 1 bei der Synthese von (R)-2-[5-(4-Fluorophenyl)-3-pyridylmethylaminomethyl]-chroman (Racemat in: EP 0 707 007 A1, Anspruch 2d) und dessen Salzen, dadurch gekennzeichnet, daß man
a) eine Verbindung der Formel II 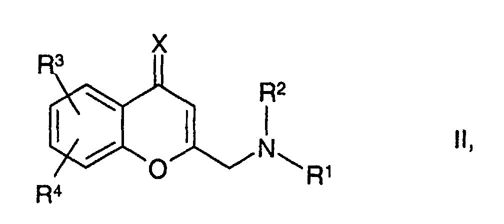 worin
   R¹ die in Anspruch 1 angegebene Bedeutung hat,
   R², R³ und R⁴ H bedeuten und X O bedeutet,
   mit Hilfe eines enantiomerenangereicherten Katalysators hydriert,
b) daß man aus dem so erhaltenen enantiomerenangereicherten Gemisch der (R)- und (S)-Verbindungen der Formel I, worin
   R¹ die in Anspruch 1 angegebene Bedeutung hat,
   R², R³ und R⁴ H bedeuten und X O bedeutet,
   die enantiomerenreine (R)-Verbindung der Formel I, worin
   R¹ die in Anspruch 1 angegebene Bedeutung hat,
   R², R³ und R⁴ H bedeuten und X O bedeutet,
   durch Kristallisation gewinnt,
   daß man
c) die enantiomerenreine (R)-Verbindung der Formel I, worin
   R¹ die in Anspruch 1 angegebene Bedeutung hat,
   R², R³ und R⁴ H bedeuten und X O bedeutet,
   anschließend wie üblich reduziert,
   daß man
d) aus der so erhaltenen (R)-Verbindung der Formel I, worin
   R¹ die in Anspruch 1 angegebene Bedeutung hat,
   R², R³ und R⁴ H bedeuten und X H,H bedeutet,
   den Rest R¹ abspaltet,
   daß man
e) das so erhaltene (R)-(Chroman-2-ylmethyl)-amin in sein Säureadditionssalz umwandelt und dieses in bekannter Weise zu (R)-2-[5-(4-Fluorophenyl)-3-pyridylmethylaminomethyl]-chroman umsetzt und gegebenenfalls in sein Säureadditionssalz umwandelt,
wobei die Gewinnung des (R)-Enantiomeren durch Kristallisation aus dem enantiomerenangereicherten (R,S)-Gemisch auch nach Stufe c) oder nach Stufe d) durchgeführt werden kann.

Gegenstand der Erfindung ist ferner die Verwendung der Verbindungen der Formel I als Zwischenprodukte zur Synthese von Arzneimitteln, die Wirkungen auf das Zentralnervensystem zeigen.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel.

### Beispiele

Experimentelle Daten (Komplexherstellung, Hydrierung, Analytik):

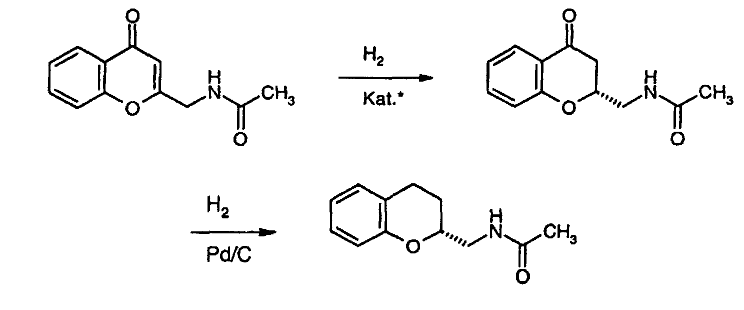

Alle Umsetzungen wurden unter inerten Bedingungen (d.h. wasserfreie und sauerstofffreie Reaktionsbedingungen) durchgeführt.

### 1. Herstellung der Katalysator-Substrat-Lösung:

### 1.1. Beispiel:

11,2 mg Rh(COD)₂OTf (Rhodium-cyclooctadien-triflat) wurden in 5 ml Methanol gelöst und auf 0 °C gekühlt. Danach wurde eine gekühlte Lösung von 1.1eq Bisphosphan (z. B. 12,6 mg (R, R)-Skewphos) in 5 ml Methanol zugesetzt. Nach 10 min Rühren bei Raumtemperatur versetzte man die Komplexlösung mit der Substratlösung bestehend aus 110 mg (2-Acetylaminomethyl)-chromen-4-on in 10 ml Methanol.

### 1.2. Beispiel:

51,4 mg [Rh(COD)Cl]₂ wurden in 4ml des Lösungsmittelgemisches Toluol/Methanol 5:1 gelöst und mit einer Lösung bestehend aus 5 ml Toluol, 1ml Methanol und 1.1 eq Bisphosphan (z. B. 130,6 mg (R)-BINAP) versetzt. 1ml dieser Katalysatorkomplex-Lösung wurde zu 510,8 mg (2-Acetylaminomethyl)-chromen-4-on, gelöst in 15 ml Toluol und 3 ml Methanol, gegeben.

### 2. Enantioselektive Hydrierung

Die zu hydrierende Katalysator-Substrat-Lösung wurde im Schutzgasgegenstrom in einen Autoklaven gefüllt. Die Schutzgasatmosphäre wurde durch mehrmaliges Spülen mit Wasserstoff (1 - 5 bar H₂-Atmosphäre) ersetzt. Die Ansätze analog 1.1. reagierten schon bei Raumtemperatur und 5 bar Wasserstoff. Die Katalysatoren analog 1.2. erbrachten bei 50°C und 80 bar Wasserstoff die besten Ergebnisse. In der Regel wurde die Hydrierung nach 15 Stunden abgebrochen.

### 3. Probennahme und Analytik

Im Schutzgasgegenstrom wurde eine Probe entnommen. Der Komplex wurde vor der Bestimmung der Enantiomerenüberschüsse durch Säulenchromatographie an Kieselgel abgetrennt (Eluent: Essigsäureethylester). Der Enantiomerenüberschuß des Hydrierprodukts wurde an chiraler HPLC-Phase bestimmt:
Säule: Daicel Chiralcel OJ (I.D. x Länge /mm : 4,6 x 250)
Eluent: n-Hexan:i-Propanol = 9:1
Flow: 0,8 ml/min (18 bar, 28 °C)
Detection: UV 250 nm
Retention: Rₜ (*R*) = 27 min; Rₜ (*S*) = 29 min

Das Einengen der rohen Hydrierlösung führte zur Ausfällung des Produkts. Man stellte eine Erhöhung des Enantiomerenüberschusses durch die fraktionierende Kristallisation fest.

### 4. Weitere Reduktion

Nachdem der vollständige Umsatz festgestellt wurde, erfolgte die Reduktion der Keto-Gruppe mittels Palladium-Kohle als Eintopf-Verfahren. Man versetzte die rohe, aus der homogenen Hydrierung resultierende Keton-Lösung mit 10-gew.-% wasserfeuchte Palladium-Kohle (z.B. auf 1 g (2-Acetylaminomethyl)-chromen-4-on 100 mg wasserfeuchte Pd/C) und 1ml Eisessig. Bei 7 bar Wasserstoffdruck und 50 °C hydrierte man 14h.

### 5. Aufarbeitung und Analytik

Die Palladium-Kohle wurde durch Filtration entfernt.
Der Enantiomerenüberschuß des Hydrierprodukts wurde an chiraler HPLC-Phase bestimmt:
Säule: Daicel Chiralcel OJ (I.D. x Länge /mm : 4,6 x 250)
Eluent: n-Hexan:i-Propanol = 9:1
Flow: 0,8 ml/min (18 bar, 28 °C)
Detection: UV 250 nm
Retention: Rₜ (*R*) = 25 min; Rₜ (*S*) = 27 min

Während der Reduktion mit Palladium-Kohle blieb der Enantiomerenüberschuß unverändert.
Das Einengen der rohen Hydrierlösung führte zur Ausfällung des Produkts. Man stellte eine Vergrößerung des Enantiomerenüberschusses durch die fraktionierende Kristallisation fest.

Enantioselektivitäten der homogenen Hydrierung:

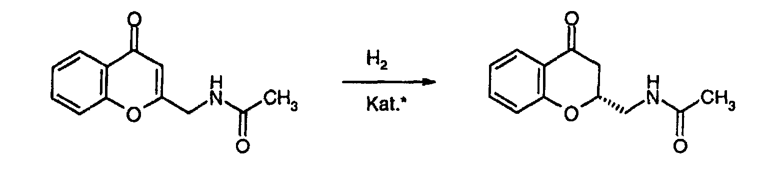

| | Elab- Nr. | Komplex: Metall-Anion-Lig (Zusatz) | Lsm | Druck | % ee |
|---|---|---|---|---|---|
| 1. | 18 | Rh-OTf-(*R*,*R*)-EtDuphos | CH₂Cl₂ | 1 | 55 S |
| 2. | 13 | Rh-OTf-(*R*,*R*)-EtDuphos | THF | 1 | 44 S |
| 3. | 14 | Rh-OTf-(*R*,*R*)-EtDuphos | MeOH | 1 | 64 S |
| 4. | 15 | Rh-OTf-(*R*,*R*)-EtDuphos | EE | 1 | 33 S |
| 5. | 6 | Rh-OTf-(*R,R*)-EtDuphos | *i*PrOH | 1 | 20 S |
| 6. | 23a | Rh-OTf-(*R*,*R*)-EtDuphos | MeOH | 1 | 34 S |
| 7. | 23b | Rh-OTf-(*R*,*R*)-EtDuphos | MeOH | 1 | 36 S |
| 8. | 23c | Rh-OTf-(*R*,*R*)-EtDuphos | MeOH | 5 | 45 S |
| 9. | 23d | Rh-OTf-(*R*,*R*)-EtDuphos | MeOH | 5 | 31 S |
| 10. | 12 | Ru-Cl₂-(*R*)-BINAP | *i*PrOH | 5 | 50 S |
| | | (AgOOCCF₃) | | | |
| 11. | 19 | Rh-ClO₄-(*S*,*S*)-Chiraphos | *i*PrOH | 1 | - |
| 12. | 20 | Rh-OTf-(*S*,*S*)-DIOP | THF | 1 | rac. |
| 13. | 20 | Rh-OTf-(*S*,*S*)-DIOP | THF | 3 | 8 R |
| 14. | 21 | Rh-OTf-(*R*,*R*)-Skewphos | THF | 1 | - |
| 15. | 22b | Rh-OTf-(*S*,*S*)-BPPM | MeOH | 1 | 7 S |
| 16. | 24a | Rh-OTf-(*R*,*S*)-BPPFOH | MeOH | 1 | 54 R |
| 17. | 24b | Rh-OTf-(*R*,*S*)-BPPFOH | MeOH | 1 | 54 R |
| 18. | 24c | Rh-OTf-(*R*,*S*)-BPPFOH | MeOH | 5 | 63 R |
| 19. | 25a | Rh-OTf-(*R*)-BINAP | MeOH | 1 | 1 R |
| 20. | 25b | Rh-OTf-(*R*)-BINAP | MeOH | 5 | rac. |
| 21. | 26a | Rh-OTf-(*S*,*S*)-Norphos | MeOH | 1 | 42 R |
| 22. | 26b | Rh-OTf-(*S*,*S*)-Norphos | MeOH | 5 | 60 R |
| 23. | 26c | Rh-OTf-(*S*,*S*)-Norphos | iPrOH | 5 | 12 R |
| 24. | 26d | Rh-OTf-(*S*,*S*)-Norphos | THF | 5 | 3 R |
| 25. | 27a | Rh-OTf-(*S*,*S*)-Norphos | MeOH | 8 | 64 R |
| 26. | 27b | Rh-Cl-(*S*,*S*)-Norphos | MeOH | 8 | 40 R |
| 27. | 27c | Rh-OTf-(*S*,*S*)-Norphos | MeOH | 30 | 65 R |
| 28. | 27d | Rh-OTf-(*S,S*)-Norphos | MeOH | 60 | 64 R |
| 29. | 28a | Rh-OTf-(*R*,*R*)-EtDUPhos | MeOH | 10 | 16 S |
| 30. | 28b | Rh-OTf-(*R*,*R*)-EtDUPhos | MeOH | 30 | 28 S |
| 31. | 29a | Rh-OTf-(*R*,*S*)-BPPFOH | MeOH | 10 | 55 R |
| 32. | 29b | Rh-OTf-(*R*,*S*)-BPPFOH | MeOH | 30 | 56 R |
| 33. | 37 | Rh-ClO₄-(*S*,*S*)-Chiraphos | MeOH | 10 | 30 R |
| 34. | 38 | Rh-OTf-(*S*,*S*)-DIOP | MeOH | 10 | rac. |
| 35. | 39 | Rh-OTf-(*R*,*R*)-Skewphos | MeOH | 10 | 46 S |
| 36. | 40 | Rh-OTf-(*S*,*S*)-BPPM | MeOH | 10 | 9 S |
| 37. | 41 | Ir-Cl-(*S*,*S*)-DIOP | MeOH | 10 | 8 R |
| 38. | 42 | Ir-Cl-(*S*,*S*)-DIOP | CH₂Cl₂ | 10 | 7 S |
| 0. | 43 | Ir-Cl-(*S,S*)-DIOP (+ I) | MeOH | 10 | - |
| 1. | 44 | Ir-Cl-(*S,S*)-DIOP (+ I) | MeOH | 30 | - |
| 2. | 45 | Ir-Cl-(*S,S*)-DIOP (+ I | MeOH | 10 | - |
| | | +CH₃COOH) | | | |
| 3. | 46 | Ir-OTf-(*S*,*S*)-DIOP | MeOH | 10 | 11R |
| 4. | 47 | Ir-OTf-(*S*,*S*)-DIOP (+ I) | MeOH | 10 | 39 R |
| 5. | 49 | Rh-OTf-(*S,S*)-Norphos | MeOH | 10, RT | 57 R |
| 6. | 50 | Rh-OTf-(*S*,*S*)-Norphos | MeOH | 10, 50°C | 60 R |
| 7. | 52 | Rh-BF₄-(*R*,*S*)-PFctB | MeOH | 10, 50°C | 33 S |
| 8. | 54 | Rh-Cl-(*R*)-BINAP | Tol:MeOH 5:1 | 80, 50°C | 91S |
| 9. | 59 | Rh-Cl-(*S*,*S*)-Norphos | Tol:MeOH 5:1 | 80, 50°C | 19 R |
| 10. | 62 | roh 59//Pd/C | Tol:MeOH 5:1 | 7, 50°C | 18 R |

## Patentansprüche

1. Chromanderivate der Formel I 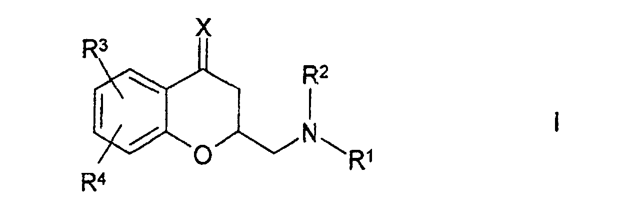 worin
R¹ Acyl mit 1-6 C-Atomen, -CO-R⁵, Phenylacetyl, Phenoxyacetyl, Methoxycarbonyl, Ethoxycarbonyl, 2,2,2-Trichlorethoxycarbonyl, tert.-Butoxycarbonyl, 2-Iodethoxycarbonyl, Carbobenzoxycarbonyl, 4-Methoxybenzyloxycarbonyl, 9-Fluorenylmethoxycarbonyl oder 4-Methoxy-2,3,6-trimethylphenyl-sulfonyl,
R² H oder Alkyl mit 1-6 C-Atomen,
R³, R⁴ jeweils unabhängig voneinander H, Alkyl mit 1-6 C-Atomen, CN, Hal oder COOR²,
R⁵ unsubstituiertes oder ein- oder zweimal durch Alkyl mit 1-6 C-Atomen, OR² oder Hal substituiertes Phenyl,
X H, H oder O,
Hal F, Cl, Br oder I
bedeuten,
sowie deren Salze,
wobei N-(Chroman-2-ylmethyl)-cyclopropancarbonsäureamid ausgeschlossen ist.

2. Enantiomere der Verbindungen der Formel I gemäß Anspruch 1.

3. Verbindungen der Formel I gemäß Anspruch 1
a) N-(4-Oxo-chroman-2-ylmethyl)-acetamid;
b) N-(Chroman-2-ylmethyl)-acetamid;
c) (S)-N-(4-Oxo-chroman-2-ylmethyl)-acetamid;
d) (R)-N-(4-Oxo-chroman-2-ylmethyl)-acetamid;
e) (S)-N-(Chroman-2-ylmethyl)-acetamid;
f) (R)-N-(Chroman-2-ylmethyl)-acetamid.
sowie deren Salze.

4. Verfahren zur Herstellung von Chromanderivaten der Formel I nach Anspruch 1 sowie von deren Salzen, worin X O bedeutet,
**dadurch gekennzeichnet, daß** man eine Verbindung der Formel II 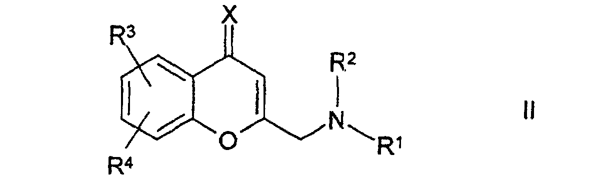 worin R¹, R², R³, R⁴ die in Anspruch 1 angegebenen Bedeutungen haben und X O bedeutet,
mit Hilfe eines enantiomerenangereicherten Katalysators hydriert.

5. Verfahren zur Herstellung von Chromanderivaten der Formel I nach Anspruch 1 sowie von deren Salzen, worin X H,H bedeutet,
**dadurch gekennzeichnet, daß** man eine Verbindung der Formel II 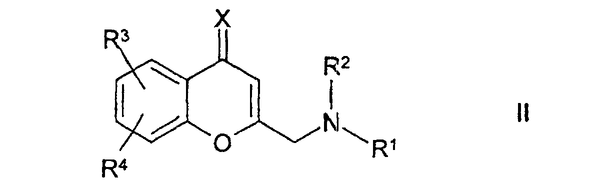 worin R¹, R², R³, R⁴ die in Anspruch 1 angegebenen Bedeutungen haben und X O bedeutet,
mit Hilfe eines enantiomerenangereicherten Katalysators hydriert, und anschließend wie üblich reduziert.

6. Verfahren zur Herstellung von Chromanderivaten der Formel I nach den Ansprüchen 4 oder 5, **dadurch gekennzeichnet, daß** es sich bei dem enantiomerenangereicherten Katalysator um einen Übergangsmetallkomplex handelt.

7. Verfahren zur Herstellung von Chromanderivaten der Formel I nach den Ansprüchen 4, 5 oder 6, **dadurch gekennzeichnet, daß** es sich bei dem Katalysator um einen Übergangsmetallkomplex, enthaltend ein Metall ausgewählt aus der Gruppe Rhodium, Iridium, Ruthenium und Palladium,
handelt.

8. Verfahren zur Herstellung von Chromanderivaten der Formel I nach den Ansprüchen 4, 5, 6 oder 7, **dadurch gekennzeichnet, daß** es sich bei dem Katalysator um einen Übergangsmetallkomplex handelt, worin das Übergangsmetall mit einem chiralen Diphosphanliganden komplexiert ist.

9. Verwendung der Verbindungen der Formel I nach Anspruch 1 als Zwischenprodukte zur Synthese von Arzneimitteln.

10. Verwendung der Verbindungen der Formel I nach Anspruch 1 als Zwischenprodukte zur Synthese von Arzneimitteln, die Wirkungen auf das Zentralnervensystem zeigen.

11. Verwendung der Verbindungen der Formel I nach Anspruch 1 bei der Synthese von
(R)-2-[5-(4-Fluorophenyl)-3-pyridylmethylaminomethyl]-chroman und dessen Salzen, **dadurch gekennzeichnet, daß** man
a) eine Verbindung der Formel II 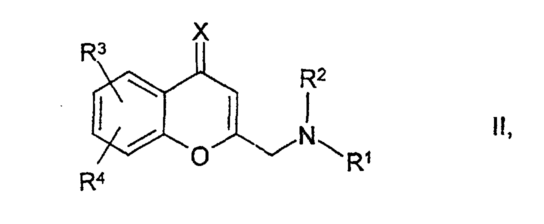 worin
R¹ die in Anspruch 1 angegebene Bedeutung hat,
R², R³ und R⁴ H bedeuten und X O bedeutet,
mit Hilfe eines enantiomerenangereicherten Katalysators hydriert,
b) daß man aus dem so erhaltenen enantiomerenangereicherten Gemisch der (R)- und (S)-Verbindungen der Formel I, worin
R¹ die in Anspruch 1 angegebene Bedeutung hat,
R², R³ und R⁴ H bedeuten und X O bedeutet,
die enantiomerenreine (R)-Verbindung der Formel I, worin
R¹ die in Anspruch 1 angegebene Bedeutung hat,
R², R³ und R⁴ H bedeuten und X O bedeutet,
durch Kristallisation gewinnt,
daß man
c) die enantiomerenreine (R)-Verbindung der Formel I, worin
R¹ die in Anspruch 1 angegebene Bedeutung hat,
R², R³ und R⁴ H bedeuten und X O bedeutet,
anschließend wie üblich reduziert,
daß man
d) aus der so erhaltenen (R)-Verbindung der Formel I, worin
R¹ die in Anspruch 1 angegebene Bedeutung hat,
R², R³ und R⁴ H bedeuten und X H,H bedeutet,
den Rest R¹ abspaltet,
daß man
e) das so erhaltene (R)-(Chroman-2-ylmethyl)-amin in sein Säureadditionssalz umwandelt und dieses in bekannter Weise zu (R)-2-[5-(4-Fluorophenyl)-3-pyridylmethylaminomethyl]-chroman umsetzt und gegebenenfalls in sein Säureadditionssalz umwandelt,
wobei die Gewinnung des (R)-Enantiomeren durch Kristallisation aus dem enantiomerenangereicherten (R,S)-Gemisch auch nach Stufe c) oder nach Stufe d) durchgeführt werden kann.

## Claims

1. Chroman derivatives of the formula I 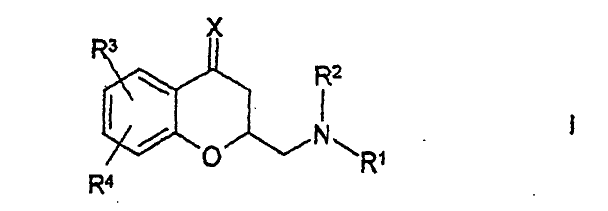 in which
R¹ is acyl having 1-6 carbon atoms, -CO-R⁵, phenylacetyl, phenoxyacetyl, methoxycarbonyl, ethoxycarbonyl, 2,2,2-trichloroethoxycarbonyl, tert-butoxycarbonyl, 2-iodoethoxycarbonyl, carbobenzoxycarbonyl, 4-methoxybenzyloxycarbonyl, 9-fluorenylmethoxycarbonyl or 4-methoxy-2,3,6-trimethylphenylsulfonyl,
R² is H or alkyl having 1-6 carbon atoms,
R³ and R⁴ are each, independently of one another, H, alkyl having 1-6 carbon atoms, CN, Hal or COOR²,
R⁵ is phenyl which is unsubstituted or monosubstituted or disubstituted by alkyl having 1-6 carbon atoms, OR² or Hal,
X is H,H or O,
Hal is F, Cl, Br or I,
and salts thereof,
where N-(chroman-2-ylmethyl)cyclopropanecarboxamide is excluded.

2. Enantiomers of the compounds of the formula I according to Claim 1.

3. Compounds of the formula I according to Claim 1
a) N-(4-oxochroman-2-ylmethyl)acetamide;
b) N-(chroman-2-ylmethyl)acetamide;
c) (S)-N-(4-oxochroman-2-ylmethyl)acetamide;
d) (R)-N-(4-oxochroman-2-ylmethyl)acetamide;
e) (S)-N-(chroman-2-ylmethyl)acetamide;
f) (R)-N-(chroman-2-ylmethyl)acetamide;
and salts thereof.

4. Process for the preparation of chroman derivatives of the formula I according to Claim 1 and salts thereof, in which X is O, **characterised in that** a compound of the formula II 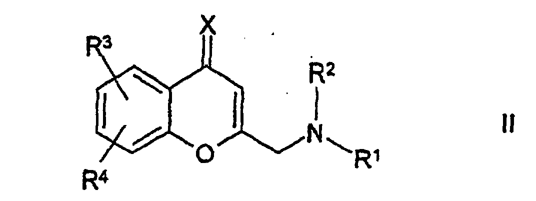 in which R¹, R², R³ and R⁴ are as defined in Claim 1, and X is O, is hydrogenated with the aid of an enantiomerically enriched catalyst.

5. Process for the preparation of chroman derivatives of the formula I according to Claim 1 and salts thereof, in which X is H,H, **characterised in that** a compound of the formula II 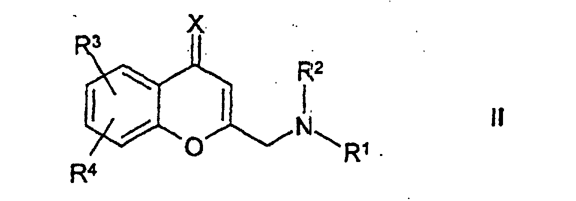 in which R¹, R², R³ and R⁴ are as defined in Claim 1, and X is O, is hydrogenated with the aid of an enantiomerically enriched catalyst and subsequently reduced in a conventional manner.

6. Process for the preparation of chroman derivatives of the formula I according to Claim 4 or 5, **characterised in that** the enantiomerically enriched catalyst is a transition-metal complex.

7. Process for the preparation of chroman derivatives of the formula I according to Claim 4, 5 or 6, **characterised in that** the catalyst is a transition-metal complex containing a metal selected from the group consisting of rhodium, iridium, ruthenium and palladium.

8. Process for the preparation of chroman derivatives of the formula I according to Claim 4, 5, 6 or 7, **characterised in that** the catalyst is a transition-metal complex in which the transition metal is complexed to a chiral diphosphine ligand.

9. Use of the compounds of the formula I according to Claim 1 as intermediates in the synthesis of medicaments.

10. Use of the compounds of the formula I according to Claim 1 as intermediates in the synthesis of medicaments which exhibit actions on the central nervous system.

11. Use of the compounds of the formula I according to Claim 1 in the synthesis of (R)-2-[5-(4-fluorophenyl)-3-pyridylmethylaminomethyl]-chroman and salts thereof, **characterised in that**
a) a compound of the formula II 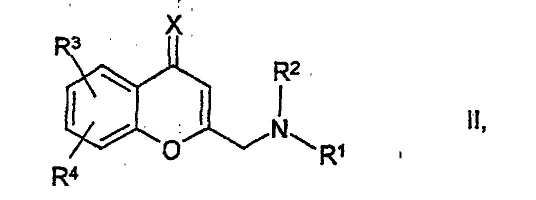 in which
R¹ is as defined in Claim 1,
R², R³ and R⁴ are H and X is O,
is hydrogenated with the aid of an enantiomerically enriched catalyst,
b) **in that** the enantiomerically pure (R) compound of the formula I in which
R¹ is as defined in Claim 1,
R², R³ and R⁴ are H and X is O,
is isolated from the resultant enantiomerically enriched mixture of the (R) and (S) compounds of the formula I in which
R¹ is as defined in Claim 1,
R², R³ and R⁴ are H and X is O,
by crystallisation,
**in that**
c) the enantiomerically pure (R) compound of the formula I in which
R¹ is as defined in Claim 1,
R², R³ and R⁴ are H and X is O,
is subsequently reduced in a conventional manner,
**in that**
d) the radical R¹ is cleaved off from the resultant (R) compound of the formula I in which
R¹ is as defined in Claim 1,
R², R³ and R⁴ are H and X is H,H,
**in that**
e) the resultant (R)-(chroman-2-ylmethyl)amine is converted into its acid-addition salt, and the latter is converted in a known manner into (R)-2-[5-(4-fluorophenyl)-3-pyridylmethylaminomethyl]chroman and, if desired, converted into its acid-addition salt,
where the isolation of the (R) enantiomer from the enantiomerically enriched (R,S) mixture by crystallisation can also be carried out after step c) or after step d).

## Revendications

1. Dérivés de chromane répondant à la formule I 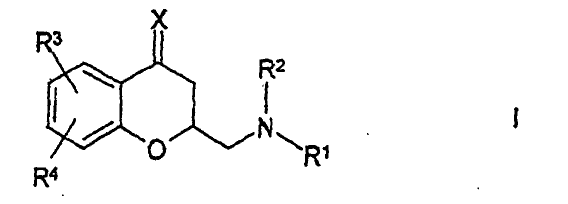 dans laquelle
R¹ représente acyle ayant de 1 à 6 atomes de carbone, -CO-R⁵, phénylacétyle, phénoxyacétyle, méthoxycarbonyle, éthoxycarbonyle, 2,2,2-trichloroéthoxycarbonyle, tert-butoxycarbonyle, 2-iodoéthoxycarbonyle, carbobenzoxycarbonyle, 4-méthoxybenzyloxycarbonyle, 9-fluorénylméthoxycarbonyle ou 4-méthoxy-2,3,6-triméthylphénylsulfonyle,
R² représente H ou alkyle ayant de 1 à 6 atomes de carbone,
R³ et R⁴ représentent chacun, indépendamment l'un de l'autre, H, alkyle ayant de 1 à 6 atomes de carbone, CN, Hal ou COOR²,
R⁵ représente phényle qui est non substitué ou monosubstitué ou disubstitué par alkyle ayant de 1 à 6 atomes de carbone, OR² ou Hal,
X représente H,H ou O,
Hal représente F, Cl, Br ou I,
et les sels de ceux-ci,
où le N-(chroman-2-ylméthyl)cyclopropanecarboxamide est exclu.

2. Enantiomères des composés répondant à la formule I selon la revendication 1.

3. Composés répondant à la formule I selon la revendication 1
a) le N-(4-oxochroman-2-ylméthyl)acétamide;
b) le N-(chroman-2-ylméthyl)acétamide;
c) le (S)-N-(4-oxochroman-2-ylméthyl)acétamide;
d) le (R)-N-(4-oxochroman-2-ylméthyl)acétamide;
e) le (S)-N-(chroman-2-ylméthyl)acétamide;
f) le (R)-N-(chroman-2-ylméthyl)acétamide;
et les sels de ceux-ci.

4. Procédé de préparation de dérivés de chromane répondant à la formule I selon la revendication 1 et ds sels de ceux-ci, dans laquelle X représente O, **caractérisé en ce qu'**un composé répondant à la formule II 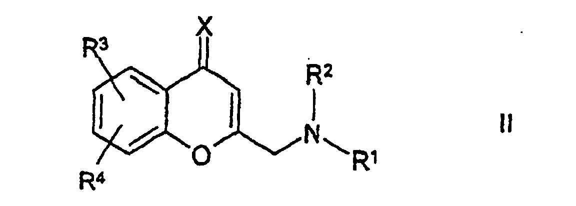 dans laquelle R¹, R², R³ et R⁴ sont tels que définis selon la revendication 1, et X représente O,
est hydrogéné à l'aide d'un catalyseur énantiomériquement enrichi.

5. Procédé de préparation de dérivés de chromane répondant à la formule I selon la revendication 1 et de sels de ceux-ci, dans laquelle X représente H,H, **caractérisé en ce qu'**un composé répondant à la formule II 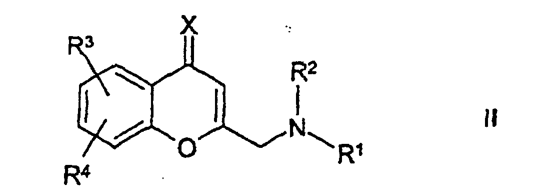 dans laquelle R¹, R², R³ et R⁴ sont tels que définis selon la revendication 1, et X représente O,
est hydrogéné à l'aide d'un catalyseur énantiomériquement enrichi et est ensuite réduit de manière classique.

6. Procédé de préparation de dérivés de chromane répondant à la formule I selon la revendication 4 ou 5, **caractérisé en ce que** le catalyseur énantiomériquement enrichi est un complexe de métal de transition.

7. Procédé de préparation de dérivés de chromane répondant à la formule I selon la revendication 4, 5 ou 6, **caractérisé en ce que** le catalyseur est un complexe de métal de transition contenant un métal choisi parmi le groupe constitué par le rhodium, l'iridium, le ruthénium et le palladium.

8. Procédé de préparation de dérivés de chromane répondant à la formule I selon la revendication 4, 5, 6 ou 7, **caractérisé en ce que** le catalyseur est un complexe de métal de transition dans lequel le métal de transition est complexé à un ligand de diphosphine chiral.

9. Utilisation des composés répondant à la formule I selon la revendication 1 comme intermédiaires dans la synthèse de médicaments.

10. Utilisation des composés répondant à la formule I selon la revendication 1 comme intermédiaires dans la synthèse de médicaments présentant des actions sur le système nerveux central.

11. Utilisation des composés répondant à la formule I selon la revendication 1 dans la synthèse du (R)-2-[5-(4-fluorophényl)-3-pyridylméthylaminométhyl]chromane et de sels de celui-ci, **caractérisée en ce que**
a) un composé répondant à la formule II 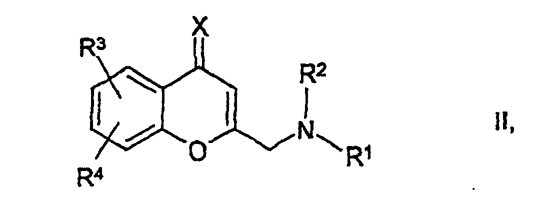 dans laquelle
R¹ est tel que défini selon la revendication 1,
R², R³ et R⁴ représentent H et X représente O,
est hydrogéné à l'aide d'un catalyseur énantiomériquement enrichi,
b) **en ce que** le composé (R) énantiomériquement pur répondant à la formule I dans laquelle
R¹ est tel que défini selon la revendication 1,
R², R³ et R⁴ représentent H et X représente O,
est isolé à partir du mélange résultant énantiomériquement enrichi
des composés (R) et (S) répondant à la formule I dans laquelle
R¹ est tel que défini selon la revendication 1,
R², R³ et R⁴ représentent H et X représente O,
par cristallisation,
**en ce que**
c) le composé (R) énantiomériquement pur répondant à la
formule I dans laquelle
R¹ est tel que défini selon la revendication 1,
R², R³ et R⁴ représentent H et X représente O,
est ensuite réduit de manière classique,
**en ce que**
d) le radical R¹ est clivé du composé (R) résultant répondant à
la formule I dans laquelle
R¹ est tel que défini selon la revendication 1,
R², R³ et R⁴ représentent H et X représente H,H,
**en ce que**
e) la (R)-(chroman-2-ylméthyl)amine résultante est convertie en son sel d'addition d'acide, et ce dernier est converti de manière connue en (R)-2-[5-(4-fluorophényl)-3-pyridylméthylaminométhyl]-chromane et, si on le désire, converti en son sel d'addition d'acide,
où l'isolement de l'énantiomère (R) par cristallisation à partir du mélange (R,S) énantiomériquement enrichi peut également être réalisé après l'étape c) ou après l'étape d).
